(19) Europäisches Patentamt
European Patent Office
Office européen des brevets

(11) EP 1 964 539 A1

(12) DEMANDE DE BREVET EUROPEEN

(43) Date de publication:
03.09.2008 Bulletin 2008/36

(21) Numéro de dépôt: 07122933.0

(22) Date de dépôt: 11.12.2007

(51) Int Cl.:
*A61K 8/02* (2006.01) *A61Q 5/06* (2006.01)
*A61K 8/40* (2006.01) *A61K 8/41* (2006.01)
*A61K 8/35* (2006.01)

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **14.12.2006 FR 0655519**

(71) Demandeur: **L'Oréal**
**75008 Paris (FR)**

(72) Inventeurs:
- **Guerin, Frédéric**
**75010 Paris (FR)**
- **Gourlaouen, Luc**
**92600 Asnières (FR)**
- **Simon, Pascal**
**94320 Thiais (FR)**

(74) Mandataire: **Dossmann, Gérard**
**Bureau D.A. Casalonga-Josse**
**Bayerstrasse 71/73**
**80335 München (DE)**

# (54) Article cosmétique pour la coloration capillaire à sec, son utilisation, procédé de mise en oeuvre dudit article

(57) La présente invention a pour objet un article cosmétique substantiellement sec destiné à la coloration capillaire comprenant
-un substrat flexible poreux,
-un ou plusieurs colorants directs ou précurseurs de colorants directs sous forme de poudre et susceptibles de colorer la matière kératinique par transfert thermique, le substrat et les colorants directs ou précurseurs de colorants directs étant fixés de manière non covalente au substrat.

La présente invention a de plus pour objet un procédé de coloration de matières kératiniques mettant en oeuvre cet article cosmétique par transfert thermique à sec. Elle concerne également l'utilisation de cet article pour la coloration de matières kératiniques notamment humaines par transfert thermique à sec.

EP 1 964 539 A1

## Description

La présente invention a pour objet un article cosmétique destiné à la coloration de matières kératiniques notamment humaines par transfert thermique à sec. Elle a de plus pour objet un procédé de coloration de matières kératiniques mettant en oeuvre cet article cosmétique par transfert thermique à sec. Elle concerne de plus l'utilisation de cet article pour la coloration de matières kératiniques notamment humaines par transfert thermique à sec.

L'invention concerne plus particulièrement le domaine de la coloration des fibres kératiniques humaines, et notamment des cheveux.

Il est connu depuis longtemps de modifier la couleur des cheveux, et en particulier de masquer les cheveux blancs.

On connaît essentiellement deux types de technologies mises en oeuvre pour la coloration des fibres kératiniques humaines.

La première méthode, appelée coloration directe ou semi-permanente, consiste à changer ou apporter de la couleur par l'application d'une molécule colorée qui pénètre par diffusion à l'intérieur de la fibre et/ou reste adsorbée à sa surface.

La seconde méthode, appelée coloration d'oxydation ou coloration permanente, consiste à changer ou apporter de la couleur en mettant en oeuvre à l'intérieur même de la fibre, une condensation oxydative de précurseurs de colorants qui sont des composés peu ou pas colorés. Après cette réaction, les colorants formés sont insolubles et sont piégés à l'intérieur de la fibre.

Les deux méthodes résumées ci-dessus permettent d'accéder à de nombreuses couleurs.

La composition de coloration se présente généralement sous des formes diverses, telles qu'une lotion, une mousse, une crème ou un gel. Cependant, la plupart de ces formes présentent l'inconvénient de couler sur le visage ou sur des zones que l'on ne souhaite pas colorer. De plus, l'utilisation de ces produits est généralement salissante, pour les vêtements, les accessoires de coiffure (peignes, serviettes....), les bacs.....

Par ailleurs, la durée du procédé de coloration est longue. En effet, après le temps de pose de la composition colorante, il est nécessaire de rincer abondamment les cheveux, voire d'effectuer un shampooing, et de les sécher.

Enfin, l'utilisation de certains colorants offrant des performances tinctoriales intéressantes sur les fibres est parfois limitée en raison de leur faible solubilité dans les supports aqueux de formulation.

Comme on peut le constater, on est toujours à la recherche de méthodes de coloration de matières kératiniques humaines qui, tout en permettant d'obtenir des colorations efficaces, ne présenteraient pas les inconvénients mentionnés ci-dessus.

Récemment, dans ses demandes FR 0502034, FR 0502030, FR 0502031, la Demanderesse a découvert qu'il était possible de colorer les cheveux à partir de poudres colorantes par transfert thermique.

Il a été démontré que certains colorants directs pouvaient colorer simplement le cheveu par transfert de colorant d'une composition sèche sur le cheveu sous l'action de la température sans utilisation d'un support véhicule liquide. Par ailleurs, toujours grâce au fait que le colorant est initialement utilisé sous forme solide, il n'y a besoin ni de rinçage, ni de shampooing et/ou de séchage des fibres kératiniques. De plus, le transfert thermique étant rapide (quelques minutes), les temps de pose sont courts.

Cette nouvelle technologie de coloration par transfert thermique à sec met en oeuvre une composition de coloration se présentant sous la forme d'un film sec polymérique contenant une dispersion d'un ou plusieurs colorants, déposé sur un substrat solide non poreux (aluminium, plastique, ...). Cette voie de fabrication des films secs par dissolution en milieu hydro-alcoolique du polymère filmogène puis dispersion des colorants, étalement de cette préparation en couche mince sur le substrat solide et enfin séchage, est longue et présente des problèmes en terme de solubilité, de formulation et de temps de séchage. De plus, les compositions se présentant sous cette forme ne sont pas faciles d'utilisation, à cause du substrat solide qui est trop rigide et peu conformable. Il est donc nécessaire de disposer d'un article plus facile à réaliser, d'utilisation plus aisée et permettant un meilleur contact avec la fibre.

La demanderesse a découvert une méthode plus simple et très facile à mettre en oeuvre pour la coloration capillaire, par transfert thermique.

Ainsi, la présente invention a pour objet un article cosmétique substantiellement sec, destiné à la coloration des matières kératiniques, de préférence les fibres kératiniques humaines, telles que des cheveux comprenant

- un substrat flexible poreux, et
- un ou plusieurs colorants directs ou précurseurs de colorants directs sous forme de poudre, susceptibles de colorer la matière kératinique par transfert thermique,

le substrat et le colorant direct ou précurseur de colorant direct étant fixés de manière non covalente sur et/ou dans le substrat,
le substrat flexible poreux étant choisi parmi des substrats fibreux non tissés et des substrats cellulaires de type mousse.

L'invention concerne également un procédé de coloration des matières kératiniques mettant en oeuvre cet article utilisant le transfert thermique à sec de ces colorants ou précurseurs de colorant.

Enfin, l'invention a également pour objet l'utilisation de cet article pour la coloration des matières kératiniques telles que les fibres kératiniques notamment les cheveux.

L'article cosmétique selon l'invention est un article sec c'est-à-dire qu'au cours de son utilisation, il n'est jamais mis en contact avec un liquide et qu'il ne comprend pas d'agent adhésif pour fixer l'agent de coloration sur le support.

Les articles de l'invention sont préparés en imprégnant le substrat flexible poreux de poudres de colorants directs ou de précurseurs de colorants directs, par voie directe et sans solvant. Cela présente l'avantage de ne pas nécessiter l'utilisation d'un support de formulation liquide pour le colorant ce qui rend la coloration particulièrement peu salissante. De plus, le fait de mettre en oeuvre directement les colorants sous leur forme solide permet d'utiliser des colorants peu solubles ou peu stables dans les milieux de teintures classiques. Cela peut contribuer à élargir encore la palette de couleurs possibles et obtenir des couleurs plus tenances.

« Substantiellement sec » signifie qu'il contient moins de 5% d'eau en poids d'eau par poids de l'article.

« fixé de manière non permanente » signifie de manière non covalente.

Les matières kératiniques humaines désignent plus particulièrement les fibres kératiniques, telles que les cheveux, les cils.

Rappelons enfin qu'à moins d'une indication différente, les bornes délimitant une gamme de valeurs font partie de cette gamme.

Le substrat flexible poreux est généralement plus connu sous le nom de lingette.

Les lingettes, notamment les lingettes cosmétiques sont généralement constituées d'un substrat en une matière d'origine naturelle ou synthétique, qui est de préférence un non-tissé, mais qui peut être aussi une mousse ou un tissu, ledit substrat étant imprégné d'une composition adaptée au but recherché. Ces lingettes sont utilisées couramment et sont appréciées pour leur côté pratique, car elles sont jetables et sont imprégnées de la quantité nécessaire et suffisante de produit nettoyant ou traitant.

Selon l'invention, les substrats flexibles poreux sont par exemple des substrats fibreux tissés ou non tissés ou des substrats cellulaires de type mousse. Le terme flexible utilisé ici fait référence à des supports fins, dont l'épaisseur est comprise entre 0,5 mm et 20 mm, de préférence entre 1 mm et 3 mm, et qui peuvent épouser facilement les contours de la surface à traiter. Ils peuvent par exemple être facilement enroulés autour d'une mèche de cheveu.

Les matériaux constituant ces substrats sont choisis de telle sorte que l'article supporte les températures nécessaires pour le transfert thermique des colorants sur le cheveu sans dégradation du substrat. On choisira donc de préférence des substrats non tissés constitués de fibres naturelles non thermofusibles, et liées entre elles grâce à des techniques, qui ne font pas appel à des liants chimiques risquant de fondre lors du transfert thermique des colorants. Comme technique, on peut citer l'hydroliage ou l'aiguilletage, le voile de fibres ayant été préalablement constitué par cardage ou par voie aérodynamique (voie dite Airlaid). Par fibres thermofusible, on entend des fibres qui gardent leur intégrité physique sous l'effet de la chaleur, et notamment au cours du transfert thermique qu'elles auront à subir dans le cadre de la présente invention. Comme fibres naturelles, on peut citer par exemple les fibres de soie, les fibres cellulosiques. Comme fibres cellulosiques, on peut citer par exemple les fibres de pulpe de bois, les fibres de coton, de jute, de lin et leur combinaison. On peut également utiliser des fibres synthétiques comme les fibres d'ester de cellulose (acétate de cellulose), la viscose, les fibres d'acide polylactique, les fibres de polyamide.

Dans le cadre de la présente invention, les colorants directs ou précurseurs de colorants directs sous forme de poudre peuvent être utilisés seuls ou en mélange avec d'autres types de poudres. La granulométrie de ces poudres est généralement comprise entre 0,1 et 100$\mu$m, plus préférentiellement, entre 0,5 et 60$\mu$m.

Ces colorants ou précurseurs de colorants directs peuvent être utilisés tels quels ou sous forme de microcapsules contenant des colorants directs ou des précurseurs de colorants directs.

Préférentiellement, les colorants directs ou les précurseurs de colorant direct mis en oeuvre dans le cadre de la présente invention sont choisis parmi les composés présentant une enthalpie de vaporisation inférieure ou égale à 200 KJ/mole.

Ces colorants peuvent être non ioniques, cationiques ou anioniques.

A titre d'exemples de colorants convenables, on peut citer les colorants acridines, acridones, anthranthrones, anthrapyrimidines, anthraquinones, azines, azoïques, azométhines, méthines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (comme par exemple les azacarbocyanines, diazacarbocyanines, diazahémicyanines, hémicyanines, tétraazacarbocyanines), diazines, dicétopyrrolopyrroles, dioxazines, diphénylamines, diphénylméthanes, dithiazines, flavonoïdes tels que flavanthrones et flavones, fluorindines, formazans, hydrazones en particulier les aryl-hydrazones, hydroxycétones, indamines, indanthrones, indigoïdes et pseudo-iondigoïdes, indophénols, indoanilines, isoindolines, isoindolines, isoindolinones, isoviolanthrones, lactones, méthines, naphthalimides, naphthanilides, naphtholactames, naphthoquinones, nitro-(hétéro)aromatiques, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes, seuls ou en mélanges.

Selon un mode de réalisation particulièrement avantageux de l'invention, le colorant direct est choisi parmi :

- un colorant azométhinique correspondant à la formule (I) suivante :

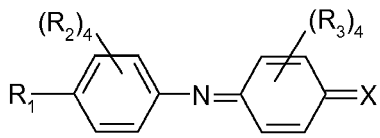

(I)

dans laquelle :

R1 représente :

un atome d'hydrogène ;
un groupement hydroxyle ;
un groupement amino ;
un groupement amino mono- ou di-substitué par un ou deux radicaux, identiques ou non, choisis parmi :

les radicaux alkyle en C1-C6 éventuellement substitués par un groupement alkyl(C1-C4)SO2NH, un groupement NH2CO- ;
un groupement alkyl(C1-C6)O2SNH-alkyl(C1-C6)NH-,
un groupement aryl(C6)alkyle(C1-C6) ;
un groupement aryle en C6 ;
un groupement alkyl(C1-C4)pipéridine ;
les radicaux portés par le groupement amino peuvent former avec l'atome d'azote auquel ils sont reliés, un hétérocycle à 5 ou 6 chaînons comprenant éventuellement un hétéroatome choisi parmi l'oxygène l'azote,

R2, identiques ou différents, représentent :

un atome d'hydrogène ;
un groupement alkyle en C1-C6, linéaire ou ramifié, éventuellement substitué par un groupement hydroxyle, alcoxy en C1-C6;
un groupement cycloalkyle en C5-C6 éventuellement substitué par un groupement hydroxyle, alcoxy en C1-C6 ;
un groupement alcoxy en C1-C6 ;
un atome d'halogène, en particulier le chlore ;
un groupement cyano ;
un groupement aryl(C6)alkyle(C1-C6) ; le groupement aryle étant éventuellement substitué par au moins un groupement groupement alkyle en C1-C6, hydroxyle, alcoxy en C1-C6 ;
un groupement aryle en C6 éventuellement substitué par au moins un groupement groupement alkyle en C1-C6, hydroxyle, alcoxy en C1-C6 ;
un groupement alkyl(C1-C6)carbonyle ;
un groupement alkyl(C1-C6)carbonylamino ;
un groupement alkyl(C1-C6)sulfonylamino ;
un groupement aminocarbonylamino ;
groupement alkyl(C1-C6)carbonylamino ;
groupement alkyl(C1-C6)sulfoamino ;
un groupement amino substitué par un ou deux radicaux, identiques ou non, choisis parmi les radicaux alkyle en C1-C6, hydroxyalkyle en C1-C6, trifluorométhyle ;

R3, identiques ou différents, représentent :

- un atome d'hydrogène,
- un atome d'halogène, en particulier à un atome de chlore,
- un groupement alkyle en C1-C6 éventuellement substitué par un groupement hydroxyle, alcoxy en C1-C6 ,

- un groupement alcoxy en C1-C6,
- un groupement alkyl(C1-C6)carbonylamino ;
- un groupement amino,
- un groupement aminoalkyl(C1-C6)carbonylamino,
- un groupement aminocarbonylamino,
- un groupement alcoxy(C1-C6)carbonylamino (aminoesteralkyle en C1-C4),
- un groupement aminocarbonyle dans lequel le groupement amino peut être mono- ou di- substitué par des radicaux identiques ou différents choisis parmi les radicaux alkyle en C1-C6, halogénoalkyle en C1-C6, aryl (C6)alkyle en C1-C6, aryle en C6, alkyl(C1-C6)carbonylamino, alkyl(C1-C6)sulfonylamino, RaCONH- où Ra est un groupement halogénoalkyle en C1-C4, un groupement aryl(C6)alkyle en C1-C6, un groupement aryle en C6, un groupement alcoxy(C1-C6)alkyle en C1-C6, un groupement hydroxyalkyle en C1-C6, un groupement cyanoalkyle en C1-C6, un groupement méthanesulfonamide, un groupement alkyle(C1-C6)aminoalkyle en C1-C6, un groupement cycloalcényle en C3-C6 substitué ou non, un groupement β-thiényl, ou l'un des groupements suivants :

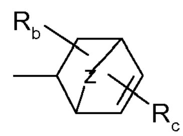

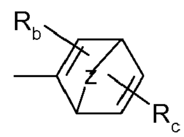

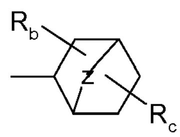

dans lesquels Rb et Rc, indépendamment l'un de l'autre, représentent un atome d'hydrogène, un atome d'halogène, une chaîne alkyle en C1-C6 et où Z représente CH2, O, S ou NRd, où Rd représente un atome d'hydrogène, une chaîne alkyle en C1-C6;
- deux radicaux R3 portés par deux atomes de carbone adjacents, peuvent former un cycle benzénique éventuellement substitué par un atome d'halogène, un groupement alkyle en C1-C6, un groupement amino, un groupement amino mono- ou di-substitué par un radical alkyle en C1-C6, hydroxyalkyle en C1-C6 ;

  X représente un atome d'oxygène, un groupement NH ;
- un colorant anthraquinonique particulier de formule (II) suivante :

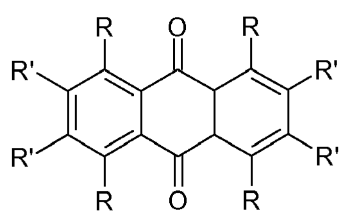

(II)

dans laquelle :

R, indépendamment les uns des autres, représentent :

un atome d'hydrogène ;
un groupement hydroxyle ;
un groupement amino ;
un groupement amino mono- ou di-substitué par un ou deux radicaux, identiques ou différents, choisis parmi

les radicaux alkyle en C1-C6, hydroxyalkyle en C1-C6, aryle en C6 éventuellement substitué par un ou plusieurs radicaux alkyle en C1-C6 ;

R', indépendamment les uns des autres, représentent :

un atome d'hydrogène ;
un atome d'halogène ;
un groupement hydroxyle ;
un groupement amino ;
un groupement amino mono- ou di-substitué par un ou deux radicaux, identiques ou différents, choisis parmi les radicaux alkyle en C1-C6, alkylamino en C1-C6, cycloalkyle en C5-C8, alcoxy (C1-C6)alkyle(C1-C6) ;
un radical aryle en C6 éventuellement substitué par un ou plusieurs groupements, identiques ou différents, choisis parmi les atomes d'halogène, les radicaux alkyle en C1-C6, alcoxy en C1-C6, alcoxy(C1-C6)alkyle(C1-C6) ;
un groupement aryl(C6)oxy ;
un groupement aryl(C6)oxy substitué par un groupement aryl(C6)-S03, le groupement aryle étant éventuellement substitué par un ou plusieurs groupements, identiques ou différents, choisis parmi les atomes d'halogène, les groupements hydroxyle, trifluorométhyle, alkyle en C1-C6, alcoxy en C1-C6, aryle en C6, aryl(C6)alkyle (C1-C4), aryl(C6)oxy, nitro, acyle en C2-C6, acyl(C2-C6)amino, alkyl(C1-C6)mercapto, thiophényle en C6, sulfonyloxy, alkyl(C1-C6)benzènesulfonyle
un groupement aryl(C6)oxy substitué par un groupement - SO2N(Ra)2 avec Ra, identiques ou non, représentant un atome d'hydrogène, un radical alkyle en C1-C6, alcényle en C2-C6, aryl(C6)alkyle(C1-C6), cycloalkyle en C5-C8, les deux radicaux Ra pouvant former avec l'atome d'azote auquel ils sont rattachés, former un hétérocycle à 5 ou 6 chaînons ;
un groupement -SRb avec Rb représentant un hétérocycle à 5 ou 6 chaînons, comprenant l'atome de soufre et comprenant éventuellement un atome d'azote, de soufre, d'oxygène ;
-COORc avec Rc représentant un groupement hydroxyalkyle en C1-C4, alkyl(C1-C4)phényle, cycloalkyle en C5-C8, carbonyloxyaryle(C6), carbonylaryle(C6) ;
-SO3-aryle(C6), le radical aryle étant éventuellement substitué par un ou plusieurs groupements, identiques ou différents, choisis parmi les atomes d'halogène, les groupements hydroxyle, trifluorométhyle, alkyle en C1-C6, alcoxy en C1-C6, aryle en C6, aryl(C6)alkyle(C1-C4), aryl(C6)oxy, nitro, acyle en C2-C6), acyl(C2-C6) amino, alkyl(C1-C6)mercapto, thiophényle, sulfonyloxy, alkyl(C1-C6)benzènesulfonyle.

- un colorant azoïque correspond à la formule (III) suivante :

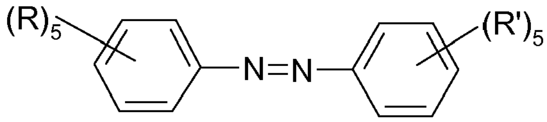

(III)

dans laquelle :

R, identiques ou non, R', identiques ou non, indépendamment les uns des autres, représentent
un atome d'hydrogène ;
une chaîne hydrocarbonée linéaire, ramifiée ou cyclique, saturée, insaturée ou aromatique, en C1-C30, éventuellement substituée par un ou plusieurs atomes d'halogène ; par un ou plusieurs groupements, identiques ou non, choisis parmi les groupements hydroxy ; nitro ; cyano ; acide carboxylique ; allyle ; halogénoallyle ; alcoxy en C1-C10 ; alkyl(C1-C4)carbonylamino en C1-C4 ; alkyl(C1-C4)aminocarbonyle ; hydrogéno-carbonylamino ; alkyl (C1-C4)sulfonylamino ; alcoxy(C1-C4)carbonylamino ; alcoxy(C1-C4)carbonyle ; alkyl(C1-C4)carbonylalcoxy(Cl-C4)carbonyle ; CF3 ; amino ; amino éventuellement substitués par un ou plusieurs radicaux identiques ou non, alkyle en C1-C10, hydroxyalkyle en C1-C10, alcoxyalkyle en C1-C10, aminoalkyle en C1-C10, (di)alkyl(C1-C10) aminoalkyle en C1-C10, cyanoalkyle en C1-C10, aryle en C6-C30, aryl(C6-C30)alkyle(C1-C4), cyclo(C4-C30)alkyle (C1-C12), alkyle(C1-C4)carbonyle, alkyle(C1-C4)carboxyalkyle(C1-C4), allyle, halogénoallyle ;
éventuellement interrompue ou rattachée au noyau aromatique par l'intermédiaire d'un ou plusieurs hétéroatomes et/ou groupement comprenant un ou plusieurs hétéroatomes choisis parmi l'oxygène, le soufre, l'azote ;
un groupement hydroxyle ; alcoxy en C1-C4 ; acide carboxylique ; amino ; amino substitué par un ou plusieurs

radicaux, identiques ou différents choisis parmi les radicaux alkyle en C1-C4, alcényle en C2-C4, cyanoalkyle en C1-C4, hydroxyalkyle en C1-C4, aryle en C6-C30, aryl(C6-C30)alkyle(C1-C4), allyle, alcoxy(C1-C4)alkyle(C1-C4), cyclo(C4-C30)alkyle(C1-C12), alkyl(C1-C4)carbonyle, alkyl(C1-C4)carboxyalkyle(C1-C4), halogénoallyle, aminoalkyle en C1-C4, (di)alkyl(C1-C4)aminoalkyle en C1-C4, alkyle(C1-C4)sulfonyle, alcoxy(C1-C4)carbonyle ; halogène ; allyle ; hydrogénocarbonyle ; trifluorométhyle ; nitro ; cyano ;
étant entendu que l'un au moins des radicaux R ou R' représente un groupement hydroxy ; nitro ; cyano ; amino ; amino éventuellement substitué par un ou plusieurs radicaux ; et
éventuellement deux radicaux R portés par deux atomes de carbone adjacents ou deux radicaux R' portés par deux atomes de carbone adjacents, peuvent former avec le cycle aromatique portant les atomes de carbone auquel chacun est rattaché, un noyau condensé de type naphtalène éventuellement substitué par un groupement aminosulfonyle ou alkyl(C1-C4)aminosulfonyle.

Selon un mode de réalisation particulier de l'invention, le radical R1 de la formula (I) représente :

un groupement hydroxyle ;
un groupement amino ;
un groupement amino mono- ou di-substitué par un ou deux radicaux, identiques ou non, choisis parmi :

les radicaux alkyle en C1-C4 éventuellement substitués par un groupement alkyl(C1-C4)SO2NH, un groupement NH2CO- ,
les radicaux portés par le groupement amino peuvent former avec l'atome d'azote auquel ils sont reliés, un hétérocycle à 5 ou 6 chaînons comprenant éventuellement un hétéroatome choisi parmi l'oxygène et l'azote.

Par ailleurs, les radicaux R2 de la formula (I), identiques ou différents, représentent plus particulièrement :

un atome d'hydrogène ;
un groupement alkyle en C1-C4, linéaire ou ramifié ;
un groupement alcoxy en C1-C4 ;
un atome d'halogène, en particulier le chlore ;

En ce qui concerne les radicaux R3 de la formula (I), ces derniers, identiques ou différents, représentent de préférence :

un atome d'hydrogène,

- un groupement alkyle en C1-C6 éventuellement substitué par un groupement hydroxyle, alcoxy en C1-C6,
- un groupement alcoxy en C1-C4,
- un groupement amino,
- un groupement alkyl(C1-C6)carbonylamino,
- un groupement alcoxy(C1-C4)carbonylamino,
- deux radicaux R3 portés par deux atomes de carbone adjacents, peuvent former un cycle benzénique éventuellement substitué par un atome d'halogène, un groupement alkyle en C1-C6, un groupement amino, un groupement amino mono- ou di-substitué par un radical alkyle en C1-C6, hydroxyalkyle en C1-C6.

De préférence, le colorant direct anthraquinonique correspond à la formule (IIa) suivante :

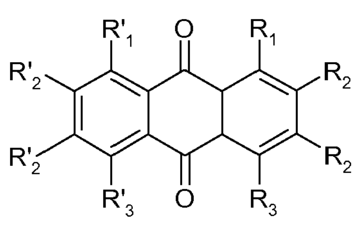

(IIa)

dans laquelle :

R1, représente un atome d'hydrogène, un groupement hydroxyle, un groupement amino, un groupement amino

mono- ou di-substitué par un ou deux radicaux, identiques ou différents, choisis parmi les radicaux alkyle en C1-C4, hydroxyalkyle en C1-C6, phényle éventuellement substitué par un ou plusieurs radicaux alkyle en C1-C4 ;
R'1 représente un atome d'hydrogène, un groupement hydroxyle, un groupement amino ;
R2, R'2, indépendamment les uns des autres, représentent
un atome d'hydrogène ;
un atome d'halogène, de préférence le chlore, le brome ;
un groupement hydroxyle ;
un groupement amino ;
un groupement amino mono- ou di-substitué par un ou deux radicaux, identiques ou différents, choisis parmi les radicaux alkyle en C1-C6, alkylamino en C1-C6, cycloalkyle en C5-C8, alcoxy (C1-C6)alkyle(C1-C6) ;
un radical aryle en C6 éventuellement substitué par un ou plusieurs groupements, identiques ou différents, choisis parmi les atomes d'halogène, les radicaux alkyle en C1-C6, alcoxy en C1-C6, alcoxy(C1-C6)alkyle(C1-C6) ;
un groupement aryl(C6)oxy ;
R3 représente un atome d'hydrogène ; un groupement hydroxyle ; un groupement amino ; un groupement amino mono- ou di-substitué par un ou deux radicaux, identiques ou différents, choisis parmi les radicaux alkyle en C1-C6, hydroxyalkyle en C1-C6, aryle en C6 éventuellement substitué par un ou plusieurs radicaux alkyle en C1-C6 ;
R'3 représente un atome d'hydrogène ; un groupement hydroxyle ; un groupement amino.

Conformément à un mode de réalisation avantageux de l'invention, le colorant direct anthraquinonique de formule (II) est choisi parmi les colorants directs suivants : le Disperse Blue 1 [2475-45-8], le Disperse Blue 3 [2475-46-9], le Disperse Blue 14 [2475-44-7], le Disperse Blue 19 [4395-65-7], le Disperse Blue 26 [3860-63-7], le Disperse Blue 56 [31810-89-6], le Disperse Orange 11 [82-28-0], le Disperse Red 60 [17418-58-5], le Disperse Violet 1 [128-95-0], le Solvent Blue 35 [17354-14-2], le Solvent Blue 59 [6994-46-3], le Solvent Green 3 [17418-58-5], la 6-méthyl-1,3,8-trihydroxyanthraquinone (Emodine) [518-82-1], la Purpurine [81-54-9], la quinalizarine [81-61-8], ainsi que leurs mélanges.

De préférence, le colorant direct azoïque correspond à la formule (IIIa) suivante :

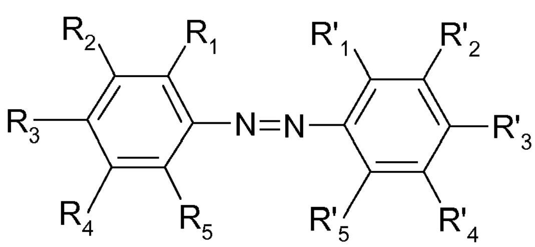

(IIIa)

dans laquelle :

R' 1 représente un atome d'hydrogène, un groupement amino ou un groupement hydroxy ;
R'2 représente un atome d'hydrogène, un atome d'halogène, un groupement acide carboxylique, un groupement alkyle en C1-C4, un groupement alcoxy en C1-C4, un groupement allyle ou un groupement aminocarbonylalkyle en C1-C4 ;
R'3 représente un atome d'hydrogène, un groupement hydroxy ou un groupement du type NR'1R'2 où R' 1 et R'2 représentent, indépendamment l'un de l'autre, un atome d'hydrogène, un groupement alkyle en C1-C4, un groupement alcényle en C2-C4, un groupement cyanoalkyle en C1-C4, un groupement hydroxyalkyle en C1-C4, un groupement aryle en C6-C30, un groupement aryl(C6-C30)alkyle(C1-C4), un groupement allyle, un groupement alcoxy(C1-C4)alkyle(C1-C4), un groupement cyclo(C4-C30)alkyle(C1-C12), un groupement alkyle(C1-C4)carbonyle, un groupement alkyle(C1-C4)carboxyalkyle(C1-C4) ou un groupement halogénoallyle, un groupement aminoalkyle en C1-C4, un groupement (di)alkyl(C1-C4)aminoalkyle en C1-C4 ;
R'2 et R'3 peuvent former avec le noyau benzénique, un noyau condensé de type naphtalène éventuellement substitué par un groupement -SO2-NHR où R représente un atome d'hydrogène ou une chaîne alkyle en C1-C4 éventuellement substituée ;
R'4 représente un atome d'hydrogène, un atome d'halogène, un groupement hydroxy, un groupement alkyle en C1-C4, un groupement alcoxy en C1-C4, un groupement alcoxy(C1-C4)alkyle(C1-C4) ;
R'5 représente un atome d'hydrogène, un atome d'halogène, un groupement hydroxy, un groupement amino, un groupement alkyle en C1-C4, un groupement alcoxy en C1-C4, un groupement alkyle(C1-C4)carbonylamino, un groupement hydrogénocarbonylamino, alkyl(C1-C4)sulfonylamino, un groupement alcoxy(C1-C4)carbonylamino

ou un groupement CF3 ;
R'4 et R'5 peuvent former avec le noyau benzénique, un noyau condensé de type naphthalène ;

R1 représente un atome d'hydrogène, un atome d'halogène, un groupement hydroxy, un groupement nitro ou un groupement cyano ;
R2 représente un atome d'hydrogène, un groupement nitro ou un groupement alkyle en C1-C4 ;
R3 représente un atome d'hydrogène, un groupement alkyle en C1-C4, un groupement alcoxy en C1-C4, un groupement cyclo(C4-C30)alkyle(C1-C12), un groupement alcényle en C2-C4, un groupement alcoxycarbonyle en C1-C4, un groupement alkyl(C1-C4)carbonyle, un groupement alcoxy(C1-C4)carbonylalcoxy(C1-C4)carbonyle, alkyl (C1-C4)carbonylalcoxy(C1-C4)carbonyle, un groupement aryle, un groupement amino, un groupement nitro, un groupement cyano ou un groupement CF3 ;
R4 représente un atome d'hydrogène ou un groupement nitro;
R5 représente un atome d'hydrogène, un atome d'halogène, un groupement acide carboxylique, un groupement hydroxy, un groupement amino, un groupement cyano ou un groupement alkyle en C1-C4 ;

étant entendu que l'un au moins des radicaux R'1 à R'5 et R1 à R5 représente un groupement hydroxy ; nitro ; cyano ; amino ; amino éventuellement substitué par un ou plusieurs radicaux.

Selon un mode de réalisation avantageux, les radicaux R'1, R'2, R1, R2 et R4 de la formule (IIIa), représentent un atome d'hydrogène.

Plus particulièrement, le radical R'3 de la formule (IIIa) représente un atome d'hydrogène, un groupement du type NR"1R"2 où R"1 et R"2 représentent, indépendamment l'un de l'autre, un atome d'hydrogène, un groupement alkyle en C1-C4, un groupement cyanoalkyle en C1-C4, un groupement hydroxyalkyle en C1-C4, un groupement aminoalkyle en C1-C4.

En ce qui concerne le radical R'4 de la formule (IIIa), celui-ci représente plus particulièrement un atome d'hydrogène.

De préférence, le radical R'5 de la formule (IIIa) représente un atome d'hydrogène, un groupement alkyle en C1-C4 .

Selon un autre mode de réalisation, les radicaux R'4 et R'5 de la formule (IIIa) forment avec le noyau benzénique, un noyau condensé de type naphthalène.

Le radical R3 de la formule (IIIa) représente plus particulièrement un groupement amino, un groupement nitro.

Le radical R5 de la formule (IIIa) représente plus particulièrement un atome d'hydrogène ; un atome d'halogène, de préférence le chlore, le fluor ; un groupement amino.

Conformément à un mode de réalisation avantageux de l'invention, le colorant direct azoïque de la formule (IIIa) est choisi parmi les colorants directs suivants : le Solvent Brown 1 [6416-57-5], le Solvent Orange 1 [2051-85-6], le Solvent Orange 7 [3118-97-6], le Solvent Yellow 2 [60-11-7], le Solvent Yellow 3 [97-56-3], le Solvent Yellow 7 [1689-82-3], le Solvent Yellow 14 [842-07-9], le Disperse Orange 1 [2581-69-3], le Disperse Orange 3 [730-40-5], le Disperse Orange 25 [31482-56-1], le Disperse Red 1 [2872-52-8], le Disperse Red 13 [3180-81-2], le Disperse Red 19 [2734-52-3], le Disperse Red 50 [12223-35-7]+[40880-51-1], le Disperse Yellow 3, le Mordant Brown 4 [6247-28-5], le Mordant Brown 6 [6247-28-5], le Mordant Brown 24 [6370-46-3], le Mordant Brown 48 [6232-53-7], le Mordant Orange 1 [2243-76-7], le Pigment Red 3 [2425-85-6], le 4-diméthylamino-2-méthylazobenzène [54-88-6], l'acide 2-(4-diéthylaminophényl azo)benzoïque [76058-33-8], la N-éthyl-N-(2-aminoéthyl)-4-(4-nitrophénylazo)aniline, la N,N-hydroxyéthyl-4-(4-nitrophénylazo)-2-méthyl aniline, la N,N-hydroxyéthyl-4-(4-aminophénylazo)-2- aniline, ainsi que leurs mélanges.

On peut aussi utiliser des précurseurs de ces colorants directs. A titre de précurseurs, on peut citer les formes leucos.

On peut aussi utiliser des colorants directs ou précurseurs de colorants directs susceptibles de réagir avec les fonctions amines présentes dans les fibres kératiniques telles que les cheveux.

Selon l'invention, le ou les colorants directs ou précurseurs de colorants directs utilisés selon l'invention représentent généralement de 0.5% à 50% en poids environ du poids total de l'article cosmétique, et plus préférentiellement de 5% à 50% en poids environ.

Les compositions de poudres de l'invention peuvent par ailleurs contenir un ou plusieurs additifs cosmétiques sous forme de poudres choisis parmi d'autres agents cosmétiques tels que des polymères (pouvant être thermofusibles), parfums, agents conservateurs, agents pH, tensioactifs, filtres, antioxydant, corps gras, silicones, acides aminés ou tout autre adjuvant utilisé habituellement en teinture de matières kératiniques.

Habituellement, la teneur en additifs dans la poudre représente pour chacun de 0,01 à 20 % ; la teneur totale en additifs, s'ils sont présents, ne dépassant pas 80 % en poids de la poudre.

Selon le procédé de préparation de l'article cosmétique mis en oeuvre, il est possible d'obtenir soit un article cosmétique imprégné dudit ou desdits colorants directs ou précuseurs de colorant peu ou pas coloré, soit d'obtenir un article substantiellement coloré.

Par « peu ou pas coloré », on entend un article dont la différence de coloration avant et après imprégnation par le ou les colorants ou le ou les précurseurs est inférieure ou égale à 10, et de préférence étant évaluée par l'écart de couleur

$$DE = \sqrt{(L_i*-L_0*)^2 + (a_i*-a_0*)^2 + (b_i*-b_0*)^2}$$

$L_i$*, $a_i$*, $b_i$* et $L_0$*, $a_0$*, $b_0$* étant respectivement les coordonnées colorimétriques dans le système La*b* de la fibre après et avant imprégnation et/ou dont la différence de chromaticité

$$DC = \sqrt{(a_i*-a_0*)^2 + (b_i*-b_0*)^2}$$

est inférieure à 5.

Par « substantiellement coloré », on entend un article pour lequel la valeur DE précédemment définie est supérieure à 5 et/ou dont la différence de chromaticité est supérieure à 5.

Dans le cadre d'articles substantiellement colorés, les colorants préférés sont les colorants directs de formule (I), (II) et (III) précédemment décrits et les précurseurs de colorant cités précédemment.

Le procédé de préparation de l'article selon l'invention peut être tout procédé d'imprégnation à sec connu. Il peut en particulier s'agir d'un procédé d'imprégnation utilisant un champ électrique alternatif, plasma à froid (0,1 à 20kV/mm) après avoir saupoudré le colorant direct ou le précurseur de colorants directs sur le substrat flexible poreux. Il n'est pas necessaire dans le cadre de l'invention de mettre en oeuvre une étape de fixation du colorants ou précurseurs de colorants.

Ce procédé est mis en oeuvre de manière à obtenir un répartition homogène du ou desdits colorants directs ou précurseurs de colorant sur et/ou dans le substrat.

Dans le cadre des articles peu ou pas colorés, cette imprégnation n'est pas suivie par une fixation thermique.

Dans le cadre des articles substantiellement colorés, l'imprégnation est suivie d'une fixation thermique dans une gamme de température comprise entre 70 et 240°C. Dans les deux cas, les subtrats imprégnés éventuellement thermofixés peuvent être recouverts d'un film protecteur en général de nature polymérique.

L'invention porte également sur un procédé de coloration capillaire mettant en oeuvre ledit article. Le procédé comprend les étapes suivantes.

Une bande de l'article cosmétique ou lingette non tissée imprégnée de poudres peut être découpée aux dimensions voulues, cette étape étant dépendante de la forme et de la dimension des lingettes disponibles.

La lingette est ensuite positionnée de part et d'autre des fibres kératiniques, par exemple une mèche de cheveux. La lingette peut par exemple entourer la mêche de cheveux, ou la prendre en sandwich.

Une source de chaleur est ensuite appliquée sur la lingette entraînant le transfert thermique du ou des colorants en surface et/ou à l'intérieur des matières kératiniques

Par ailleurs, par transfert thermique, on entend au sens de la présente invention, l'application de chaleur sur la composition sèche mise en contact des matières kératiniques à traiter ou à proximité de ces dernières. Cette chaleur est obtenue au moyen d'une source à une température plus particulièrement comprise 100 et 500°C, avantageusement entre 130 et 250°C, et encore plus avantageusement entre 140 et 220°C.

La source de chaleur peut être apportée de manière classique, comme par exemple un sèche-cheveux, un casque de coiffeur, un fer à lisser, un fer à friser, un système laser impulsionnel ou non (un rayonnement lumineux UV, visible ou infra-rouge énergétique), un système de pointes chauffantes, etc...

De préférence, cette source de chaleur est mise au contact avec l'ensemble de la lingette et des matières kératiniques à traiter.

La source de chaleur est appliquée sur la liguette, comme par exemple un fer à lisser, pendant un temps généralement compris entre 1 seconde et 5 minutes, et préférentiellement entre 30 secondes et 3 minutes. Plus la température est importante, moins la durée de traitement sera longue.

Sans être tenu par une quelconque théorie, l'un des mécanismes possibles pour la coloration des matières kératiniques passe par une étape de vaporisation ou de sublimation du colorant direct présent dans la composition sèche selon l'invention.

Des exemples concrets mais non limitatifs de l'invention vont maintenant être présentés.

EXEMPLES

Composition :

Les lingettes de l'invention sont réalisées à partir de deux substrats non tissés 100% viscose (fibres non thermo fusibles) suivants :

- Non tissé fin peu épais et de faible grammage : V 68 L Aquadim : Tharreau
- Non tissé épais plus épais de plus fort grammage : VI 41 17C : Tharreau,

et imprégnées des colorants Disperse Blue 3 ou Disperse Red 60 par la technique plasma à froid à un taux d'imprégnation des poudres de colorant (par rapport au poids du non tissé) qui est respectivement de 10% pour le non tissé épais et de 15% pour le non tissé fin.

| | |
|---|---|
| Disperse Blue 3 | 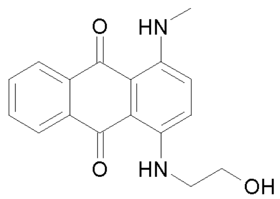 |
| Disperse Red 60 | 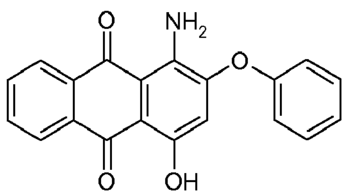 |

Procédé de coloration :

Une bande de non tissé imprégné de colorant est découpée aux dimensions de 5 x 9cm, pour être ensuite positionnée de part et d'autre d'une mèche de cheveux 90% blancs naturels préalablement étalée.

Le fer à lisser est ensuite appliqué pendant 5 minutes à 180°C.

Résultats :

Les échantillons de non tissés permettent un transfert de couleur sur cheveux, rouge fuchsia pour le Disperse Red 60 et bleu cyan pour le Disperse Blue 3.

## Revendications

1. Article cosmétique substantiellement sec destiné à la coloration des matières kératiniques, comprenant

    - un substrat flexible poreux, et
    - un ou plusieurs colorants directs ou précurseurs de colorants directs sous forme de poudre, susceptibles de colorer la matière kératinique par transfert thermique,

    le substrat et le ou les colorants directs ou précurseurs de colorants directs étant fixés de manière non covalente sur et/ou dans le substrat,,
    le substrat flexible poreux étant choisi parmi des substrats fibreux non tissés et des substrats cellulaires de type mousse.

2. Article cosmétique selon la revendication 1, **caractérisé en ce que** l'article cosmétique est obtenu par un procédé d'impregnation à sec.

**3.** Article cosmétique selon la revendication 2, **caractérisé en ce que** l'article cosmétique est obtenu par un procédé d'impregnation à sec utilisant un champ électrique alternatif.

**4.** Article cosmétique selon l'une quelconque des revendications précédentes, **caractérisé en ce que** l'épaisseur du substrat flexible poreux est comprise entre 0,5 mm et 20 mm, de préférence entre 1 mm et 3 mm.

**5.** Article cosmétique selon l'une quelconque des revendications précédentes, **caractérisé en ce que** le substrat flexible poreux est choisi parmi des substrats non tissés constitués de fibres naturelles non thermofusibles.

**6.** Article cosmétique selon l'une quelconque des revendications précédentes, **caractérisé en ce que** le substrat flexible poreux est choisi parmi les fibres de soie, les fibres cellulosiques. les fibres de pulpe de bois, les fibres de coton, de jute, de lin et leur combinaison, les fibres d'ester de cellulose, la viscose, les fibres d'acide polylactique, les fibres de polyamide.

**7.** Article cosmétique selon l'une quelconque des revendications précédentes, **caractérisé en ce que** la granulométrie de la poudre de colorants directs ou de précurseurs de colorant direct est comprise entre 0,1 et 100$\mu$m, et encore plus préférentiellement, entre 0,5 et 60$\mu$m.

**8.** Article cosmétique selon l'une quelconque des revendications précédentes, **caractérisé en ce que** le colorant direct ou le précurseur de colorant direct est choisi parmi les composés présentant une enthalpie de vaporisation inférieure ou égale à 200 KJ/mole.

**9.** Article cosmétique selon l'une quelconque des revendications précédentes, **caractérisé en ce qu'**il comprend un ou plusieurs colorants directs ou précurseurs de colorant direct non ioniques, cationiques ou anioniques.

**10.** Article cosmétique selon la revendication 9, **caractérisé en ce** le colorant direct est choisi parmi les colorants acridines, acridones, anthranthrones, anthrapyrimidines, anthraquinones, azines, azoïques, azométhines, méthines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines, diazines, dicétopyrrolopyrroles, dioxazines, diphénylamines, diphénylméthanes, dithiazines, flavonoïdes, fluorindines, formazans, hydrazones, hydroxycétones, indamines, indanthrones, indigoïdes et pseudo-iondigoïdes, indophénols, indoanilines, isoindolines, isoindolines, isoindolinones, isoviolanthrones, lactones, méthines, naphthalimides, naphthanilides, naphtholactames, naphthoquinones, nitro-(hétéro)aromatiques, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes, seuls ou en mélanges.

**11.** Article cosmétique selon la revendication 9 ou 10, **caractérisé en ce** le colorant direct est choisi parmi :

- un colorant azométhinique correspondant à la formule (I) suivante :

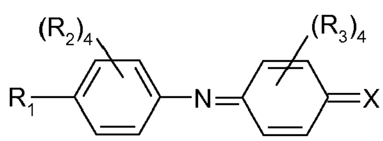

(I)

Dans laquelle :

R1 représente :

un atome d'hydrogène ;
un groupement hydroxyle ;
un groupement amino ;
un groupement amino mono- ou di-substitué par un ou deux radicaux, identiques ou non, choisis parmi :

les radicaux alkyle en C1-C6 éventuellement substitués par un groupement alkyl(C1-C4)SO2NH, un

groupement NH2CO- ;
un groupement alkyl(C1-C6)O2SNH-alkyl(C1-C6)NH-,
un groupement aryl(C6)alkyle(C1-C6) ;
un groupement aryle en C6 ;
un groupement alkyl(C1-C4)pipéridine ;
les radicaux portés par le groupement amino peuvent former avec l'atome d'azote auquel ils sont reliés, un hétérocycle à 5 ou 6 chaînons comprenant éventuellement un hétéroatome choisi parmi l'oxygène l'azote,

R2, identiques ou différents, représentent :

un atome d'hydrogène ;
un groupement alkyle en C1-C6, linéaire ou ramifié, éventuellement substitué par un groupement hydroxyle, alcoxy en C1-C6;
un groupement cycloalkyle en C5-C6 éventuellement substitué par un groupement hydroxyle, alcoxy en C1-C6 ;
un groupement alcoxy en C1-C6 ;
un atome d'halogène, en particulier le chlore ;
un groupement cyano ;
un groupement aryl(C6)alkyle(C1-C6) ; le groupement aryle étant éventuellement substitué par au moins un groupement groupement alkyle en C1-C6, hydroxyle, alcoxy en C1-C6 ;
un groupement aryle en C6 éventuellement substitué par au moins un groupement groupement alkyle en C1-C6, hydroxyle, alcoxy en C1-C6 ;
un groupement alkyl(C1-C6)carbonyle ;
un groupement alkyl(C1-C6)carbonylamino ;
un groupement alkyl(C1-C6)sulfonylamino ;
un groupement aminocarbonylamino ;
groupement alkyl(C1-C6)carbonylamino ;
groupement alkyl(C1-C6)sulfoamino ;
un groupement amino substitué par un ou deux radicaux, identiques ou non, choisis parmi les radicaux alkyle en C1-C6, hydroxyalkyle en C1-C6, trifluorométhyle ;

R3, identiques ou différents, représentent :

- un atome d'hydrogène,
- un atome d'halogène, en particulier à un atome de chlore,
- un groupement alkyle en C1-C6 éventuellement substitué par un groupement hydroxyle, alcoxy en C1-C6 ,
- un groupement alcoxy en C1-C6,
- un groupement alkyl(C1-C6)carbonylamino ;
- un groupement amino,
- un groupement aminoalkyl(C1-C6)carbonylamino,
- un groupement aminocarbonylamino,
- un groupement alcoxy(C1-C6)carbonylamino (aminoesteralkyle en C1-C4),
- un groupement aminocarbonyle dans lequel le groupement amino peut être mono- ou di- substitué par des radicaux identiques ou différents choisis parmi les radicaux alkyle en C1- C6, halogénoalkyle en C1-C6, aryl (C6) alkyle en C1- C6, aryle en C6, alkyl (C1- C6) carbonylamino, alkyl (C1- C6) sulfonylamino, RaCONH- où Ra est un groupement halogénoalkyle en C1- C4, un groupement aryl (C6) alkyle en C1-C6, un groupement aryle en C6, un groupement alcoxy (C1- C6) alkyle en C1- C6, un groupement hydroxyalkyle en C1- C6, un groupement cyanoalkyle en C1- C6, un groupement méthanesulfonamide, un groupement alkyle (C1- C6) aminoalkyle en C1- C6, un groupement cycloalcényle en C3- C6 substitué ou non, un groupement β- thiényl, ou l'un des groupements suivants :

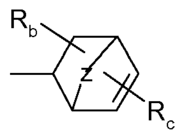

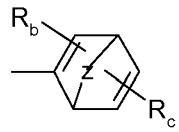

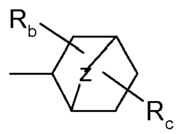

dans lesquels Rb et Rc, indépendamment l'un de l'autre, représentent un atome d'hydrogène, un atome d'halogène, une chaîne alkyle en C1-C6 et où Z représente CH2, O, S ou NRd, où Rd représente un atome d'hydrogène, une chaîne alkyle en C1-C6;

- deux radicaux R3 portés par deux atomes de carbone adjacents, peuvent former un cycle benzénique éventuellement substitué par un atome d'halogène, un groupement alkyle en C1-C6, un groupement amino, un groupement amino mono- ou di-substitué par un radical alkyle en C1-C6, hydroxyalkyle en C1-C6 ;
X représente un atome d'oxygène, un groupement NH ;
- un colorant anthraquinonique particulier de formule (II) suivante :

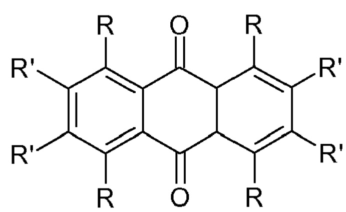
(II)

dans laquelle :

R, indépendamment les uns des autres, représentent :

un atome d'hydrogène ;
un groupement hydroxyle ;
un groupement amino ;
un groupement amino mono- ou di-substitué par un ou deux radicaux, identiques ou différents, choisis parmi les radicaux alkyle en C1-C6, hydroxyalkyle en C1-C6, aryle en C6 éventuellement substitué par un ou plusieurs radicaux alkyle en C1-C6 ;

R', indépendamment les uns des autres, représentent :

un atome d'hydrogène ;
un atome d'halogène ;
un groupement hydroxyle ;
un groupement amino ;
un groupement amino mono- ou di-substitué par un ou deux radicaux, identiques ou différents, choisis parmi les radicaux alkyle en C1-C6, alkylamino en C1-C6, cycloalkyle en C5-C8, alcoxy (C1-C6)alkyle(C1-C6) ;
un radical aryle en C6 éventuellement substitué par un ou plusieurs groupements, identiques ou différents, choisis parmi les atomes d'halogène, les radicaux alkyle en C1-C6, alcoxy en C1-C6, alcoxy(C1-C6)alkyle(C1-C6) ;
un groupement aryl(C6)oxy ;
un groupement aryl(C6)oxy substitué par un groupement aryl(C6)-SO3, le groupement aryle étant éventuellement substitué par un ou plusieurs groupements, identiques ou différents, choisis parmi les atomes d'halogène, les groupements hydroxyle, trifluorométhyle, alkyle en C1-C6, alcoxy en C1-C6, aryle en C6, aryl(C6)alkyle(C1-C4), aryl(C6)oxy, nitro, acyle en C2-C6, acyl(C2-C6)amino, alkyl(C1-C6)mercapto, thiophényle en C6, sulfonyloxy, alkyl(C1-C6)benzènesulfonyle
un groupement aryl(C6)oxy substitué par un groupement - SO2N(Ra)2 avec Ra, identiques ou non, représentant un atome d'hydrogène, un radical alkyle en C1-C6, alcényle en C2-C6, aryl(C6)alkyle(C1-C6), cycloalkyle en C5-C8, les deux radicaux Ra pouvant former avec l'atome d'azote auquel ils sont rattachés, former un hétérocycle à 5 ou 6 chaînons ;
un groupement -SRb avec Rb représentant un hétérocycle à 5 ou 6 chaînons, comprenant l'atome de soufre et comprenant éventuellement un atome d'azote, de soufre, d'oxygène ;
-COORc avec Rc représentant un groupement hydroxyalkyle en C1-C4, alkyl(C1-C4)phényle, cycloalkyle en C5-C8, carbonyloxyaryle(C6), carbonylaryle(C6) ;
-SO3-aryle(C6), le radical aryle étant éventuellement substitué par un ou plusieurs groupements, identiques ou différents, choisis parmi les atomes d'halogène, les groupements hydroxyle, trifluorométhyle, alkyle en C1-C6, alcoxy en C1-C6, aryle en C6, aryl(C6)alkyle(C1-C4), aryl(C6)oxy, nitro, acyle en C2-C6), acyl(C2-C6)amino, alkyl(C1-C6)mercapto, thiophényle, sulfonyloxy, alkyl(C1-C6)benzènesulfonyle ;

- un colorant azoïque correspond à la formule (III) suivante :

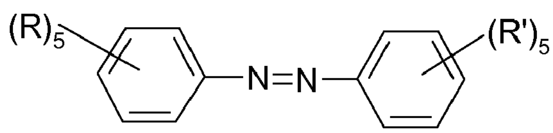
(III)

dans laquelle :
R, identiques ou non, R', identiques ou non, indépendamment les uns des autres, représentent
un atome d'hydrogène ;
une chaîne hydrocarbonée linéaire, ramifiée ou cyclique, saturée, insaturée ou aromatique, en C1-C30, éventuellement substituée par un ou plusieurs atomes d'halogène ; par un ou plusieurs groupements, identiques ou non, choisis parmi les groupements hydroxy ; nitro ; cyano; acide carboxylique ; allyle ; halogénoallyle ; alcoxy en C1-C10 ; alkyl(C1-C4)carbonylamino en C1-C4 ; alkyl(C1-C4)aminocarbonyle ; hydrogénocarbonylamino ; alkyl(C1-C4)sulfonylamino ; alcoxy(C1-C4)carbonylamino ; alcoxy(C1-C4)carbonyle ; alkyl (C1-C4)carbonylalcoxy(C1-C4)carbonyle ; CF3 ; amino ; amino éventuellement substitués par un ou plusieurs radicaux identiques ou non, alkyle en C1-C10, hydroxyalkyle en C1-C10, alcoxyalkyle en C1-C10, aminoalkyle en C1-C10, (di)alkyl(C1-C10)aminoalkyle en C1-C10, cyanoalkyle en C1-C10, aryle en C6-C30, aryl(C6-C30) alkyle(C1-C4), cyclo(C4-C30)alkyle(C1-C12), alkyle(C1-C4)carbonyle, alkyle(C1-C4)carboxyalkyle(C1-C4), allyle, halogénoallyle ;
éventuellement interrompue ou rattachée au noyau aromatique par l'intermédiaire d'un ou plusieurs hétéroatomes et/ou groupement comprenant un ou plusieurs hétéroatomes choisis parmi l'oxygène, le soufre, l'azote ;
un groupement hydroxyle ; alcoxy en C1-C4 ; acide carboxylique ; amino ; amino substitué par un ou plusieurs radicaux, identiques ou différents choisis parmi les radicaux alkyle en C1-C4, alcényle en C2-C4, cyanoalkyle en C1-C4, hydroxyalkyle en C1-C4, aryle en C6-C30, aryl(C6-C30)alkyle(C1-C4), allyle, alcoxy(C1-C4)alkyle (C1-C4), cyclo(C4-C30)alkyle(C1-C12), alkyl(C1-C4)carbonyle, alkyl(C1-C4)carboxyalkyle(C1-C4), halogénoallyle, aminoalkyle en C1-C4, (di)alkyl(C1-C4)aminoalkyle en C1-C4, alkyle(C1-C4)sulfonyle, alcoxy(C1-C4) carbonyle ; halogène ; allyle ; hydrogénocarbonyle ; trifluorométhyle ; nitro ; cyano ;
étant entendu que l'un au moins des radicaux R ou R' représente un groupement hydroxy ; nitro ; cyano ; amino ; amino éventuellement substitué par un ou plusieurs radicaux ; et
éventuellement deux radicaux R portés par deux atomes de carbone adjacents ou deux radicaux R' portés par deux atomes de carbone adjacents, peuvent former avec le cycle aromatique portant les atomes de carbone auquel chacun est rattaché, un noyau condensé de type naphtalène éventuellement substitué par un groupement aminosulfonyle ou alkyl(C1-C4)aminosulfonyle.

**12.** Article cosmétique selon la revendication 11, **caractérisé en ce** le radical R1 de la formula (I) représente :

un groupement hydroxyle ;
un groupement amino ;
un groupement amino mono- ou di-substitué par un ou deux radicaux, identiques ou non, choisis parmi :

les radicaux alkyle en C1-C4 éventuellement substitués par un groupement alkyl(C1-C4)SO2NH, un groupement NH2CO- ,
les radicaux portés par le groupement amino peuvent former avec l'atome d'azote auquel ils sont reliés, un hétérocycle à 5 ou 6 chaînons comprenant éventuellement un hétéroatome choisi parmi l'oxygène et l'azote.

**13.** Article cosmétique selon la revendication 11 ou 12, **caractérisé en ce** les radicaux R2 de la formula (I), identiques ou différents, représentent plus particulièrement :

un atome d'hydrogène ;
un groupement alkyle en C1-C4, linéaire ou ramifié ;
un groupement alcoxy en C1-C4 ;
un atome d'halogène, en particulier le chlore.

**14.** Article cosmétique selon l'une quelconque des revendications 11 à 13, **caractérisé en ce** les radicaux R3 de la formula (I), ces derniers, identiques ou différents, représentent de préférence :

un atome d'hydrogène,

- un groupement alkyle en C1-C6 éventuellement substitué par un groupement hydroxyle, alcoxy en C1-C6,
- un groupement alcoxy en C1-C4,
- un groupement amino,
- un groupement alkyl(C1-C6)carbonylamino,
- un groupement alcoxy(C1-C4)carbonylamino,
- deux radicaux R3 portés par deux atomes de carbone adjacents, peuvent former un cycle benzénique éventuellement substitué par un atome d'halogène, un groupement alkyle en C1-C6, un groupement amino, un groupement amino mono- ou di-substitué par un radical alkyle en C1-C6, hydroxyalkyle en C1-C6.

**15.** Article cosmétique selon la revendication 11, **caractérisé en ce** le colorant direct anthraquinonique correspond à la formule (IIa) suivante :

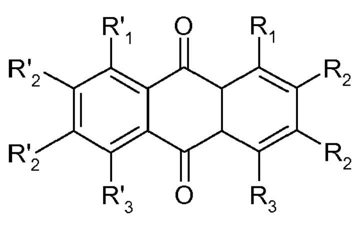

(IIa)

dans laquelle :

R1, représente un atome d'hydrogène, un groupement hydroxyle, un groupement amino, un groupement amino mono- ou di-substitué par un ou deux radicaux, identiques ou différents, choisis parmi les radicaux alkyle en C1-C4, hydroxyalkyle en C1-C6, phényle éventuellement substitué par un ou plusieurs radicaux alkyle en C1-C4 ;
R'1 représente un atome d'hydrogène, un groupement hydroxyle, un groupement amino ;
R2, R'2, indépendamment les uns des autres, représentent
un atome d'hydrogène ;
un atome d'halogène, de préférence le chlore, le brome ;
un groupement hydroxyle ;
un groupement amino ;
un groupement amino mono- ou di-substitué par un ou deux radicaux, identiques ou différents, choisis parmi les radicaux alkyle en C1-C6, alkylamino en C1-C6, cycloalkyle en C5-C8, alcoxy (C1-C6)alkyle(C1-C6) ;

un radical aryle en C6 éventuellement substitué par un ou plusieurs groupements, identiques ou différents, choisis parmi les atomes d'halogène, les radicaux alkyle en C1-C6, alcoxy en C1-C6, alcoxy(C1-C6)alkyle(C1-C6) ;

un groupement aryl(C6)oxy ;

R3 représente un atome d'hydrogène ; un groupement hydroxyle ; un groupement amino ; un groupement amino mono- ou di-substitué par un ou deux radicaux, identiques ou différents, choisis parmi les radicaux alkyle en C1-C6, hydroxyalkyle en C1-C6, aryle en C6 éventuellement substitué par un ou plusieurs radicaux alkyle en C1-C6 ;

R'3 représente un atome d'hydrogène ; un groupement hydroxyle ; un groupement amino.

**16.** Article cosmétique selon la revendication 15, **caractérisé en ce** le colorant direct anthraquinonique de formule (IIa) est choisi parmi les colorants directs suivants : le Disperse Blue 1 [2475-45-8], le Disperse Blue 3 [2475-46-9], le Disperse Blue 14 [2475-44-7], le Disperse Blue 19 [4395-65-7], le Disperse Blue 26 [3860-63-7], le Disperse Blue 56 [31810-89-6], le Disperse Orange 11 [82-28-0], le Disperse Red 60 [17418-58-5], le Disperse Violet 1 [128-95-0], le Solvent Blue 35 [17354-14-2], le Solvent Blue 59 [6994-46-3], le Solvent Green 3 [17418-58-5], la 6-méthyl-1,3,8-trihydroxyanthraquinone (Emodine) [518-82-1], la Purpurine [81-54-9], la quinalizarine [81-61-8], ainsi que leurs mélanges.

**17.** Article cosmétique selon la revendication 11, **caractérisé en ce** le colorant direct azoïque correspond à la formule (IIIa) suivante :

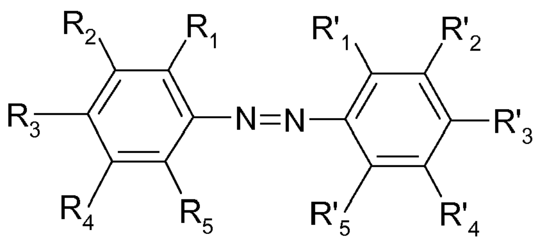

(IIIa)

dans laquelle :

R' 1 représente un atome d'hydrogène, un groupement amino ou un groupement hydroxy ;

R'2 représente un atome d'hydrogène, un atome d'halogène, un groupement acide carboxylique, un groupement alkyle en C1-C4, un groupement alcoxy en C1-C4, un groupement allyle ou un groupement aminocarbonylalkyle en C1-C4 ;

R'3 représente un atome d'hydrogène, un groupement hydroxy ou un groupement du type NR'1R'2 où R'1 et R'2 représentent, indépendamment l'un de l'autre, un atome d'hydrogène, un groupement alkyle en C1-C4, un groupement alcényle en C2-C4, un groupement cyanoalkyle en C1-C4, un groupement hydroxyalkyle en C1-C4, un groupement aryle en C6-C30, un groupement aryl(C6-C30)alkyle(C1-C4), un groupement allyle, un groupement alcoxy(C1-C4)alkyle(C1-C4), un groupement cyclo(C4-C30)alkyle(C1-C12), un groupement alkyle (C1-C4)carbonyle, un groupement alkyle(C1-C4)carboxyalkyle(C1-C4) ou un groupement halogénoallyle, un groupement aminoalkyle en C1-C4, un groupement (di)alkyl(C1-C4)aminoalkyle en C1-C4 ;

R'2 et R'3 peuvent former avec le noyau benzénique, un noyau condensé de type naphtalène éventuellement substitué par un groupement -SO2-NHR où R représente un atome d'hydrogène ou une chaîne alkyle en C1-C4 éventuellement substituée ;

R'4 représente un atome d'hydrogène, un atome d'halogène, un groupement hydroxy, un groupement alkyle en C1-C4, un groupement alcoxy en C1-C4, un groupement alcoxy(C1-C4)alkyle(C1-C4) ;

R'5 représente un atome d'hydrogène, un atome d'halogène, un groupement hydroxy, un groupement amino, un groupement alkyle en C1-C4, un groupement alcoxy en C1-C4, un groupement alkyle(C1-C4)carbonylamino, un groupement hydrogénocarbonylamino, alkyl(C1-C4)sulfonylamino, un groupement alcoxy(C1-C4)carbonylamino ou un groupement CF3 ;

R'4 et R'5 peuvent former avec le noyau benzénique, un noyau condensé de type naphthalène ;

R1 représente un atome d'hydrogène, un atome d'halogène, un groupement hydroxy, un groupement nitro ou un groupement cyano ;

R2 représente un atome d'hydrogène, un groupement nitro ou un groupement alkyle en C1-C4 ;

R3 représente un atome d'hydrogène, un groupement alkyle en C1-C4, un groupement alcoxy en C1-C4, un

groupement cyclo(C4-C30)alkyle(C1-C12), un groupement alcényle en C2-C4, un groupement alcoxycarbonyle en C1-C4, un groupement alkyl(C1-C4)carbonyle, un groupement alcoxy(C1-C4)carbonylalcoxy(C1-C4)carbonyle, alkyl(C1-C4)carbonylalcoxy(C1-C4)carbonyle, un groupement aryle, un groupement amino, un groupement nitro, un groupement cyano ou un groupement CF3 ;
R4 représente un atome d'hydrogène ou un groupement nitro ;
R5 représente un atome d'hydrogène, un atome d'halogène, un groupement acide carboxylique, un groupement hydroxy, un groupement amino, un groupement cyano ou un groupement alkyle en C1-C4 ;

étant entendu que l'un au moins des radicaux R' 1 à R'5 et R1 à R5 représente un groupement hydroxy ; nitro ; cyano ; amino ; amino éventuellement substitué par un ou plusieurs radicaux.

**18.** Article cosmétique selon la revendication 17, **caractérisé en ce** les radicaux R'1, R'2, R1, R2, R4 de la formule (IIIa), représentent un atome d'hydrogène.

**19.** Article cosmétique selon la revendication 17, **caractérisé en ce** le radical R'3 de la formule (IIIa) représente un atome d'hydrogène, un groupement du type NR" 1R"2 où R"1 et R"2 représentent, indépendamment l'un de l'autre, un atome d'hydrogène, un groupement alkyle en C1-C4, un groupement cyanoalkyle en C1-C4, un groupement hydroxyalkyle en C1-C4, un groupement aminoalkyle en C1-C4.

**20.** Article cosmétique selon l'une quelconque des revendications 17 à 19, **caractérisé en ce** le radical R'4 de la formule (IIIa) représente un atome d'hydrogène.

**21.** Article cosmétique selon l'une quelconque des revendications 17 à 20, **caractérisé en ce** le radical R'5 de la formule (IIIa) représente un atome d'hydrogène, un groupement alkyle en C1-C4.

**22.** Article cosmétique selon l'une quelconque des revendications 17 à 19, **caractérisé en ce** les radicaux R'4 et R'5 de la formule (IIIa) forment avec le noyau benzénique, un noyau condensé de type naphthalène.

**23.** Article cosmétique selon l'une quelconque des revendications 17 à 22, **caractérisé en ce** le radical R3 de la formule (IIIa) représente un groupement amino, un groupement nitro.

**24.** Article cosmétique selon l'une quelconque des revendications 17 à 23, **caractérisé en ce** le radical R5 de la formule (IIIa) représente un atome d'hydrogène ; un atome d'halogène, de préférence le chlore, le fluor ; un groupement amino.

**25.** Article cosmétique selon l'une quelconque des revendications 17 à 24, **caractérisé en ce** le colorant direct azoïque de la formule (IIIa) est choisi parmi les colorants directs suivants : le Solvent Brown 1 [6416-57-5], le Solvent Orange 1 [2051-85-6], le Solvent Orange 7 [3118-97-6], le Solvent Yellow 2 [60-11-7], le Solvent Yellow 3 [97-56-3], le Solvent Yellow 7 [1689-82-3], le Solvent Yellow 14 [842-07-9], le Disperse Orange 1 [2581-69-3], le Disperse Orange 3 [730-40-5], le Disperse Orange 25 [31482-56-1], le Disperse Red 1 [2872-52-8], le Disperse Red 13 [3180-81-2], le Disperse Red 19 [2734-52-3], le Disperse Red 50 [12223-35-7]+[40880-51-1], le Disperse Yellow 3, le Mordant Brown 4 [6247-28-5], le Mordant Brown 6 [6247-28-5], le Mordant Brown 24 [6370-46-3], le Mordant Brown 48 [6232-53-7], le Mordant Orange 1 [2243-76-7], le Pigment Red 3 [2425-85-6], [le 4-diméthylamino-2-méthylazobenzène [54-88-6], l'acide 2-(4-diéthylaminophényl azo)benzoïque [76058-33-8], la N-éthyl-N-(2-aminoéthyl)-4-(4-nitrophénylazo)aniline, la N,N-hydroxyéthyl-4-(4-nitrophénylazo)-2-méthyl aniline, la N,N-hydroxyéthyl-4-(4-aminophénylazo)-2- aniline, ainsi que leurs mélanges.

**26.** Article cosmétique selon l'une quelconque des revendications précédentes, **caractérisé en ce que** le ou les colorants directs ou précurseurs de colorants directs représentent de 0.5% à 50% en poids du poids total de l'article cosmétique, et plus préférentiellement de 5% à 50% en poids.

**27.** Procédé de coloration de matières kératiniques **caractérisé en ce que** l'on positionne l'article cosmétique tel que défini à l'une quelconque des revendications 1 à 26 sur ou à proximité des matières kératiniques, et **en ce que** l'on applique une source de chaleur entraînant le transfert thermique du ou des colorants sur ou dans les matières kératiniques.

**28.** Procédé selon la revendication précédente, **caractérisé en ce que** la source de chaleur est à une température comprise entre 100 et 500˚C, de préférence entre 130 et 250˚C.

**29.** Procédé selon la revendication 27 ou 28, **caractérisé en ce que** la durée d'application de la source de chaleur est comprise entre 1 seconde et 5 minutes, et préférentiellement entre 30 secondes et 3 minutes.

**30.** Utilisation de l'article cosmétique tel que défini à l'une quelconque des revendications 1 à 26, pour la coloration des fibres kératiniques.

Office européen des brevets

# RAPPORT DE RECHERCHE EUROPEENNE

Numéro de la demande
EP 07 12 2933

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 90/06745 A (COPELAN JAMES [US]; COPELAN PHOEBE [US]) 28 juin 1990 (1990-06-28)<br>* revendications 1,19 *<br>* page 7, alinéa F *<br>* page 16, alinéa X *<br>* page 17, alinéa XIV *<br>* page 25, ligne 1,2 *<br>* page 25, dernier alinéa *<br>----- | 1,2,4-6, 9,10 | INV.<br>A61K8/02<br>A61Q5/06<br>A61K8/40<br>A61K8/41<br>A61K8/35 |
| D,Y | WO 2006/089806 A (OREAL [FR]; PLOS GREGORY [JP]; GOURLAOUEN LUC [FR]; BAZIN DE BEZONS JE) 31 août 2006 (2006-08-31)<br>* page 2, ligne 20 - ligne 24 *<br>* page 5, ligne 5 - page 10, ligne 31 *<br>* page 11, ligne 1 - ligne 14 *<br>* page 12, ligne 11 - ligne 17 *<br>* revendications *<br>----- | 1-10, 16-30 | |
| Y | EP 0 890 326 A2 (AUGUSTIN JEAN [FR]) 13 janvier 1999 (1999-01-13)<br>* colonne 1, ligne 3 - ligne 7 *<br>* colonne 1, ligne 32 - colonne 2, ligne 4 *<br>* revendications *<br>----- | 1-30 | **DOMAINES TECHNIQUES RECHERCHES (IPC)**<br>A61Q<br>A61K<br>A45D |
| D,Y | WO 2006/089807 A (OREAL [FR]; PLOS GREGORY [JP]; GOURLAOUEN LUC [FR]; BAZIN DE BEZONS JE) 31 août 2006 (2006-08-31)<br>* page 2, ligne 20 - ligne 25 *<br>* page 3, ligne 21 - ligne 27 *<br>* page 5, ligne 1 - page 9, ligne 16 *<br>* revendications *<br>-----<br>-/-- | 1-14, 26-30 | |

Le présent rapport a été établi pour toutes les revendications

| Lieu de la recherche | Date d'achèvement de la recherche | Examinateur |
|---|---|---|
| Berlin | 28 juillet 2008 | Pelli Wablat, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

Office européen des brevets

# RAPPORT DE RECHERCHE EUROPEENNE

Numéro de la demande
EP 07 12 2933

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,Y | WO 2006/089809 A (OREAL [FR]; PLOS GREGORY [JP]; GOURLAOUEN LUC [FR]; BAZIN DE BEZONS JE) 31 août 2006 (2006-08-31)<br>* page 2, ligne 20 - ligne 25 *<br>* page 3, ligne 23 - ligne 30 *<br>* page 5, ligne 11 - page 8, ligne 31 *<br>* page 10, ligne 11 - ligne 17 *<br>* revendications *<br>----- | 1-11,15, 16,26-30 | |
| A | DE 10 2006 008542 A1 (HENKEL KGAA [DE]) 7 décembre 2006 (2006-12-07)<br>* page 2, alinéa 7 - alinéa 8 *<br>* revendications *<br>----- | 1-27 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 28 juillet 2008 | Pelli Wablat, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................
& : membre de la même famille, document correspondant

5

EPO FORM 1503 03.82 (P04C02)

## ANNEXE AU RAPPORT DE RECHERCHE EUROPEENNE RELATIF A LA DEMANDE DE BREVET EUROPEEN NO. EP 07 12 2933

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-07-2008

| Document brevet cité au rapport de recherche | | Date de publication | Membre(s) de la famille de brevet(s) | | Date de publication |
|---|---|---|---|---|---|
| WO 9006745 | A | 28-06-1990 | AUCUN | | |
| WO 2006089806 | A | 31-08-2006 | EP 1855643 | A1 | 21-11-2007 |
| | | | FR 2882521 | A1 | 01-09-2006 |
| | | | US 2008148496 | A1 | 26-06-2008 |
| EP 0890326 | A2 | 13-01-1999 | DE 69814746 | D1 | 26-06-2003 |
| | | | FR 2769186 | A1 | 09-04-1999 |
| WO 2006089807 | A | 31-08-2006 | EP 1855636 | A1 | 21-11-2007 |
| | | | FR 2882519 | A1 | 01-09-2006 |
| | | | US 2008120792 | A1 | 29-05-2008 |
| WO 2006089809 | A | 31-08-2006 | EP 1855638 | A1 | 21-11-2007 |
| | | | US 2008168608 | A1 | 17-07-2008 |
| DE 102006008542 | A1 | 07-12-2006 | AUCUN | | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 0502034
- FR 0502030
- FR 0502031